# EUROPEAN PATENT APPLICATION

(11) **EP 3 120 776 A1**
(43) Date of publication of application: **25.01.2017**
(21) Application number: 15840589.4
(22) Date of filing: 21.04.2015
(51) Int. Cl.: A61B 10/04, A61B 1/00

(54) **TREATMENT INSTRUMENT INSERTION AID**

(30) Priority: 09.09.2014 JP 2014183513
(71) Applicant: Olympus Corporation, Tokyo 192-8507 (JP)
(72) Inventor: HASHIGUCHI Toshihiko, Tokyo 192-507 (JP)
(74) Representative: Schicker, Silvia
(86) International application number: PCT/JP2015/062064
(87) International publication number: WO 2016/038924

(57) **Abstract**

A treatment instrument insertion assisting tool 1 is provided with: a connection portion 2 configured to be connected to a channel of a tool inserted into a subject; an introduction port 3 for a treatment instrument inserted into the channel via the connection portion 2; a cylindrical guiding portion 4 tapered from the introduction port 3 to the connection portion 2; and a plurality of recess portions 5 provided on an outer circumferential edge of the introduction port 3, each of the recess portions 5 having such a shape that gradually increases in width from the connection portion 2 side toward the introduction port 3 side and that is recessed toward the connection portion 2 side.

## Description

### Technical Field

The present invention relates to a treatment instrument insertion assisting tool for causing a treatment instrument inserted into a channel of a tool inserted into a subject.

### Background Art

A configuration and a method for inserting a treatment instrument into a channel of a rigid endoscope to perform treatment inside a subject by the treatment instrument are well known.

As an example, a configuration and a method are well known in which a biopsy needle is inserted into a channel of an ultrasound probe for acquiring an ultrasound image of an inside of a prostate gland via an urethra or a rigid endoscope for acquiring an optical image of the inside of the prostate gland, and the biopsy needle is caused to project from a distal end of the channel to perform a biopsy procedure for the prostate gland.

Here, when the biopsy needle is elongatedly formed, a distal end side of a needle of the biopsy needle easily vibrates in a state that an operation portion of the biopsy needle is grasped by a surgeon before the biopsy needle is inserted into the channel.

There is a problem that, when the distal end side of the needle vibrates, it is difficult for the surgeon who grasps the ultrasound probe by one hand and grasps the operation portion of the biopsy needle by the other hand to perform the work of inserting the distal end side of the biopsy needle into an opening of the channel of the rigid endoscope by himself.

Therefore, conventionally, a method of having an assistant grasp the distal end side of the needle and having the assistant guide the distal end side of the needle to the opening of the channel has been used. In this method, however, there are problems that the assistant is required to have a high-level insertion technique, that much treatment time is required, and that treatment cost increases because of increase in the number of treatment personnel.

Therefore, in Japanese Patent Application Laid-Open Publication No. 2003-79565, a configuration is disclosed in which a connection portion of a cylindrical treatment instrument insertion assisting tool configured to cause a treatment instrument to be easily inserted is connected to the opening of the channel of the rigid endoscope.

Here, in a case of inserting the needle of the biopsy needle into the opening of the channel of a tool inserted into a subject using the treatment instrument insertion assisting tool disclosed in Japanese Patent Application Laid-Open Publication No. 2003-79565, a method is conceivable in which the distal end side of the biopsy needle is caused to be inserted into the channel via the treatment instrument insertion assisting tool by causing a part of the vibrating needle to come into contact with an outer circumferential edge of an introduction port for the treatment instrument insertion assisting tool to stop the vibration of the distal end side of the needle first, pulling the distal end of the biopsy needle slightly rearward of the introduction port in that state, and, after that, pressing the distal end of the biopsy needle into the treatment instrument insertion assisting tool via the introduction port.

However, there is a possibility that, when the part of the needle is caused to come into contact with the outer circumferential edge of the introduction port in order to stop vibration of the distal end side of the needle, the part of the needle moves, sliding along the outer circumferential edge of the introduction port, and slips off from the treatment instrument insertion assisting tool because the outer circumferential edge of the introduction port has a circular arc shape.

Note that, since a configuration in which a latching portion configured to cause a part of the treatment instrument inserted into the channel to be latched is provided on the outer circumferential edge of the introduction port is disclosed in Japanese Patent Application Laid-Open Publication No. 2003-79565, a configuration is also conceivable in which the vibration is stopped by causing a part of the needle to come into contact with the latching portion, and sliding of the needle along the outer circumferential edge of the introduction port is prevented by the latching portion.

It is, however, common to perform a biopsy procedure for a prostate gland for a plurality of positions of the prostate gland by changing a position of the biopsy needle projecting from the distal end of the channel while causing the ultrasound probe, that is, the treatment instrument assisting tool fixed to the ultrasound probe to rotate by a predetermined angle in a circumferential direction. Therefore, there is a problem that a position of the latching portion is displaced in the circumferential direction each time the treatment instrument insertion assisting tool is rotated, and it becomes difficult to perform the work of causing a part of the needle to come into contact with the latching portion.

The present invention is for solving the above problem, and an object is to provide a treatment instrument insertion assisting tool having such a configuration that, at time of inserting a treatment instrument into a channel of a tool inserted into a subject to perform treatment at a plurality of positions, a surgeon can easily cause the treatment instrument to be inserted into the channel of the tool inserted into a subject by himself.

### Disclosure of Invention

### Means for Solving the Problem

A treatment instrument insertion assisting tool according to an aspect of the present invention is provided with: a connection portion configured to be connected to a channel of a tool inserted into a subject; an introduction port for a treatment instrument to be inserted into the channel via the connection portion; a cylindrical guiding portion tapered from the introduction port to the connection portion; and a plurality of recess portions provided on an outer circumferential edge of the introduction port, each of the recess portions having such a shape that gradually increases in width from the connection portion side toward the introduction port side and that is recessed toward the connection portion side.

### Brief Description of the Drawings

Fig. 1 is a perspective view showing a treatment instrument insertion assisting tool of a first embodiment together with an assisting tool connecting portion of a tool inserted into a subject;
Fig. 2 is a perspective view showing the tool inserted into a subject to which the treatment instrument insertion assisting tool of Fig. 1 is connected;
Fig. 3 is a perspective view showing a state that a needle of a biopsy needle is inserted into a channel of an insertion portion of the tool inserted into a subject of Fig. 2 via the treatment instrument insertion assisting tool, and a distal end side of the biopsy needle projects from a distal end of the channel;
Fig. 4 is a perspective view showing a state that the insertion portion of the tool inserted into a subject of Fig. 2 is inserted into a channel of an ultrasound probe;
Fig. 5 is a perspective view showing a state that the needle of the biopsy needle is inserted into the channel of the insertion portion of the tool inserted into a subject of Fig. 4 via the treatment instrument insertion assisting tool, and the distal end side of the biopsy needle projects from a distal end of the channel of the ultrasound probe;
Fig. 6 is a perspective view showing a modification of the treatment instrument insertion assisting tool in which recess portions of Fig. 1 are configured in an even number and provided at unequal intervals, together with the assisting tool connecting portion of the tool inserted into a subject;
Fig. 7 is a perspective view showing a treatment instrument insertion assisting tool of a second embodiment together with the assisting tool connecting portion of the tool inserted into a subject;
Fig. 8 is a perspective view showing the tool inserted into a subject to which the treatment instrument insertion assisting tool of Fig. 7 is connected;
Fig. 9 is a diagram schematically showing positions of puncture with a needle in a case of performing a biopsy procedure for a prostate gland at twelve positions; and
Fig. 10 is a perspective view showing a modification of the plurality of recess portions of the treatment instrument insertion assisting tool together with the assisting tool connecting portion of the tool inserted into a subject.

### Best Mode for Carrying Out the Invention

Embodiments of the present invention will be described below with reference to drawings. Note that it should be noticed that drawings are schematic, and a relationship between thickness and width of each member, a thickness ratio among respective members and the like are different from actual ones. Among the drawings, portions having a different dimension relationship or ratio are, of course, included.

### (First embodiment)

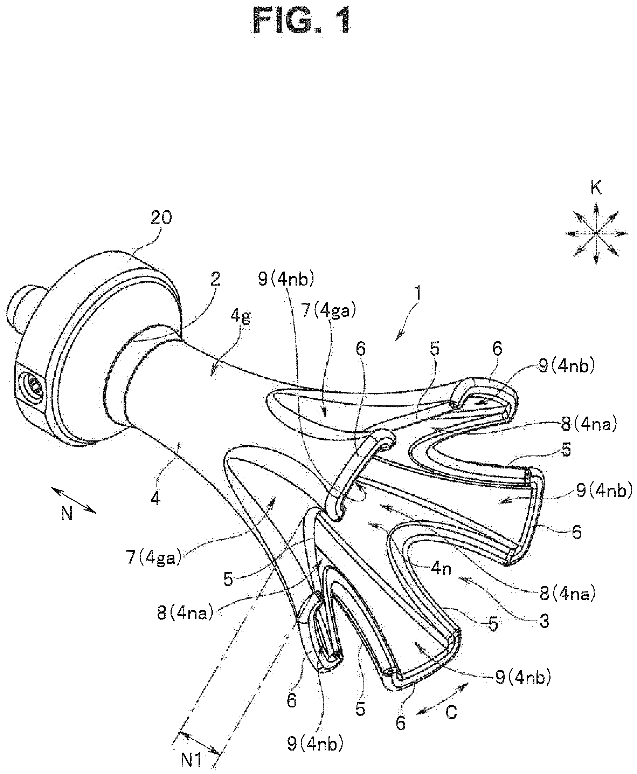

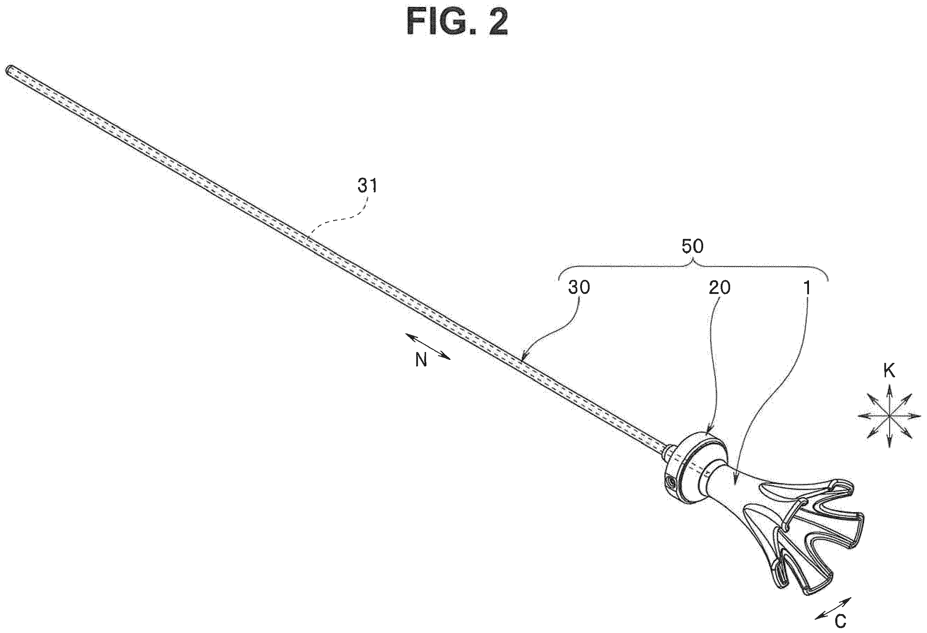

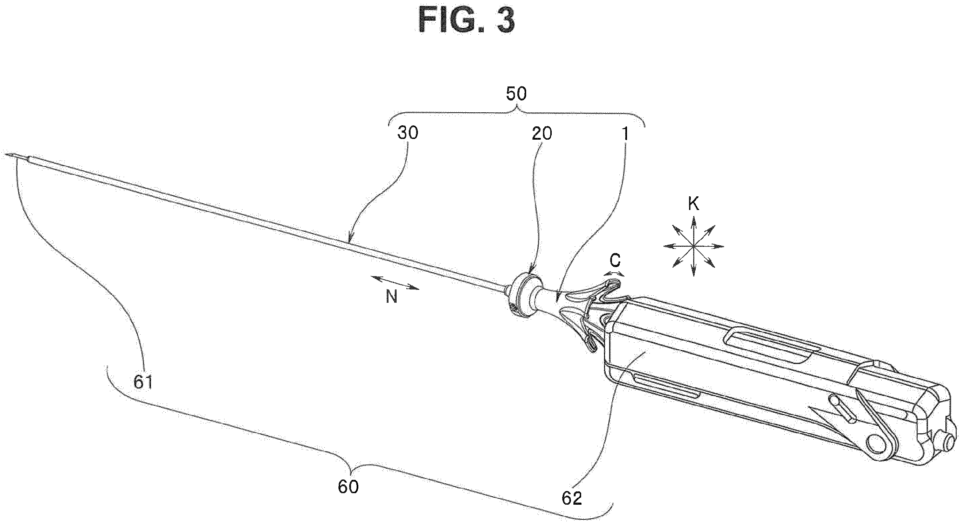

Fig. 1 is a perspective view showing a treatment instrument insertion assisting tool of the present embodiment together with an assisting tool connecting portion of a tool inserted into a subject; Fig. 2 is a perspective view showing the tool inserted into a subject to which the treatment instrument insertion assisting tool of Fig. 1 is connected; and Fig. 3 is a perspective view showing a state that a needle of a biopsy needle is inserted into a channel of an insertion portion of the tool inserted into a subject of Fig. 2 via the treatment instrument insertion assisting tool, and a distal end side of the biopsy needle projects from a distal end of the channel.

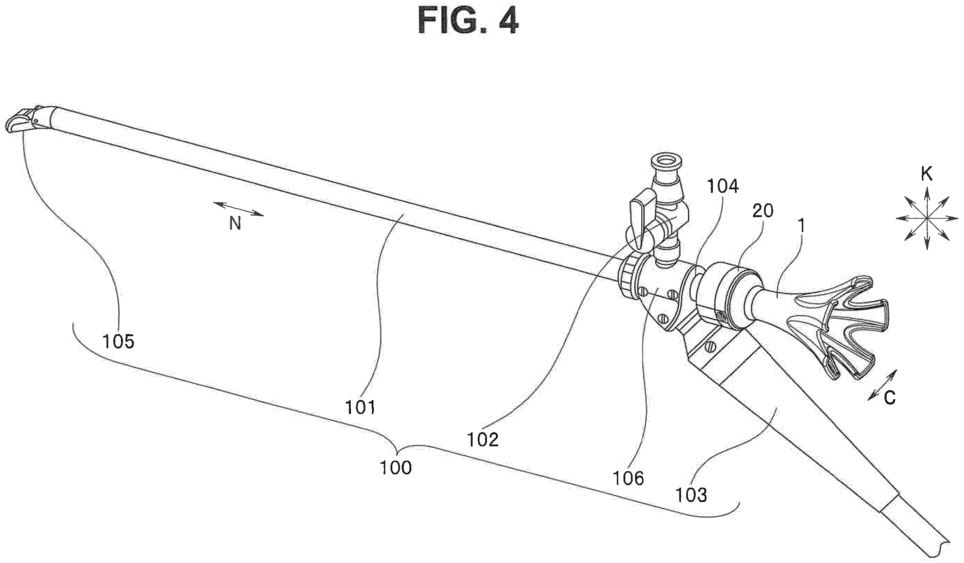

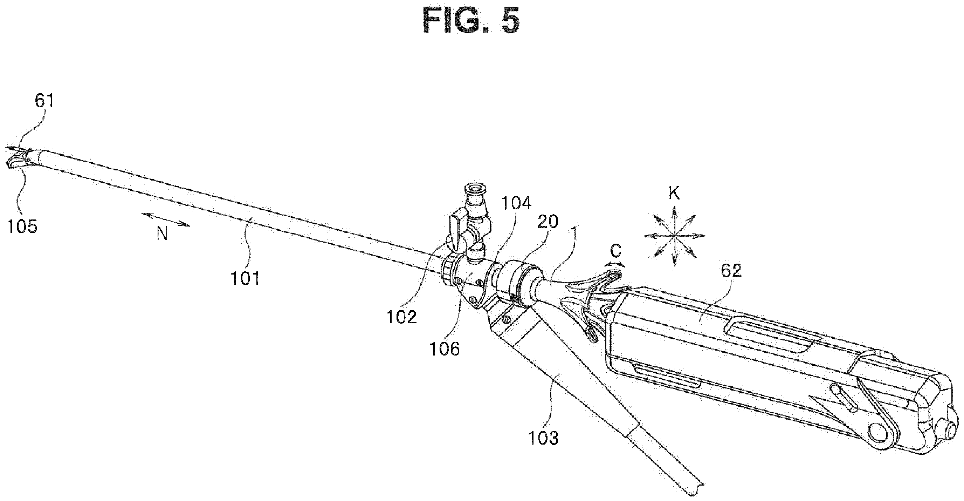

Further, Fig. 4 is a perspective view showing a state that the insertion portion of the tool inserted into a subject of Fig. 2 is inserted into a channel of an ultrasound probe; and Fig. 5 is a perspective view showing a state that the needle of the biopsy needle is inserted into the channel of the insertion portion of the tool inserted into a subject of Fig. 4 via the treatment instrument insertion assisting tool, and the distal end side of the biopsy needle projects from a distal end of the channel of the ultrasound probe.

As shown in Fig. 1, a treatment instrument insertion assisting tool 1 has a substantially conical shape having a connection portion 2, an introduction port 3, and a tubular guiding portion 4 tapered from the introduction port 3 to the connection portion 2.

Note that, in the present embodiment, the treatment instrument insertion assisting tool 1 has a substantially conical shape, for example, formed with a uniform thickness with resin.

The connection portion 2 is connected to a channel of a tool inserted into a subject. Note that, in the present embodiment, the tool inserted into a subject will be described with an obturator 50 shown in Figs. 2 and 3 as an example. The tool inserted into a subject in the present invention, however, is not limited to an obturator, but medical equipment inserted into a subject, such as a urethroscope, a rigid endoscope and a flexible ultrasound endoscope, is applicable.

More specifically, as shown in Figs. 2 and 3, the connection portion 2 is connected to a proximal end of a channel 31 provided in an insertion portion 30 of an obturator 50, in a direction N, by being connected to an assisting tool connecting portion 20 of the obturator 50.

The introduction port 3 is an opening configured to cause a treatment instrument to be inserted into the channel 31 via the connection portion 2 to be introduced into the treatment instrument insertion assisting tool 1.

Note that, in the present embodiment, the treatment instrument will be described, with a biopsy needle 60 provided with a needle 61 and an operation portion 62 connected to a proximal end of the needle 61 in the direction N as an example as shown in Fig. 3.

The needle 61 is, for example, for performing a biopsy procedure for a prostate gland and is formed to be elongated in the direction N and have a small diameter. As an example, the needle 61 is formed with a length of about 200 mm in the direction N and with a diameter of 1.2 mm to 1.3 mm.

Note that the needle 61 may be attachable to and detachable from the operation portion 62. Note that, though the needle 61 has a double structure in order to collect tissue after puncture, description of a detailed configuration will be omitted because it is well known.

Further, it is because of a purpose of making it easy to guide a needle tip of the needle 61 to come into contact with an inner circumferential face 4n, to the connection portion 2 by a wedge effect due to a slope of the inner circumferential face 4n that the guiding portion 4 has a substantially conical shape.

The operation portion 62 is a part grasped by a surgeon. Additionally, for example, at time of performing a biopsy procedure for a prostate gland, the operation portion 62 is operated when the needle 61 is caused to project forward in the direction N to puncture the prostate gland. Note that, since a detailed configuration of the biopsy needle 60 is well known, further description will be omitted.

Returning to Fig. 1, a plurality of recess portions 5 each of which has such a shape that gradually increases in width from the connection portion 2 side toward the introduction port 3 side in the direction N and that is recessed toward the connection portion 2 side by N1 are provided on an outer circumferential edge of the introduction port 3 for the treatment instrument insertion assisting tool 1.

Note that, as such a shape that gradually increases in width from the connection portion 2 side toward the introduction port 3 side in the direction N and that is recessed toward the connection portion 2 side, a semi-elliptical shape as shown Fig. 1 is given. The shape, however, is not limited to that, and a triangular shape is also possible.

More specifically, the plurality of recess portions 5 are provided in an odd number of three or more on the outer circumferential edge of the introduction port 3 and provided at equal intervals in a circumferential direction C of the introduction port 3.

Note that, in the present embodiment, the plurality of recess portions 5 are five recess portions provided on the outer circumferential edge of the introduction port 3 as an example as shown in Fig. 1.

The plurality of recess portions 5 are such that, at time of inserting the needle 61 into the channel 31 of the obturator 50 via the treatment instrument insertion assisting tool 1, stops vibration of a distal end side of the needle 61 by a part of the needle 61 being caused to come into contact with an edge portion of each recess portion 5, and prevents the needle 61 from slipping off in the circumferential direction C along the outer circumferential edge of the introduction port 3.

As the part to be caused to come into contact with the edge portion of each recess portion 5, a proximal end side of the needle 61 is preferable. This is because it is easy to cause the proximal end side of the needle 61 to come into contact with the edge portion of each recess portion 5 since there is little vibration on the proximal end side of the needle 61.

Further, the plurality of recess portions 5 are such that causes the distal end side of the needle 61 whose vibration has been stopped by the plurality of recess portions 5 to be dropped and introduced into the treatment instrument insertion assisting tool 1, accompanying rearward movement of the needle 61 in the direction N.

Further, on an outer circumferential face 4g of the guiding portion 4, a plurality of first guide faces 7, each of which is recessed toward an inner side of the guiding portion 4 in a diameter direction K, are formed on parts 4ga in communication with the plurality of recess portions 5 in the direction N.

Note that each first guide face 7 is formed to have a predetermined length along the direction N and has such a recess shape of inclining toward a center of the first guide face 7 in the circumferential direction C.

Further, the central position of each first guide face 7 in the circumferential direction C corresponds to the central position of each recess portion 5 having a semi-elliptical shape as described above.

The plurality of first guide faces 7 are such that, at the time of inserting the needle 61 into the channel 31 of the obturator 50 via the treatment instrument insertion assisting tool 1, stop vibration of the distal end side of the needle 61 by being contacted by a part of the needle 61, in conjunction with the plurality of recess portions 5, prevent the needle 61 from slipping off along the circumferential direction C on the outer circumferential face 4g of the guiding portion 4, due to having the recess shape, and, furthermore, guide the distal end side of the needle 61 to a recess portion 5.

Further, on parts 4na in communication with the plurality of recess portions 5 in the direction N on the inner circumferential face 4n of the guiding portion 4, a plurality of second guide faces 8 for the needle 61 starting from the plurality of first guide faces 7 and raised toward the inner side in the diameter direction K are formed.

Furthermore, on parts 4nb in communication with a plurality of raised parts 6 described later in the direction N on the inner circumferential face 4n of the guiding portion 4, a plurality of third guide faces 9 configured to guide the needle 61 from the introduction port 3 to the connection portion 2 are formed.

The plurality of second guide faces 8 are formed to have a certain length along the direction N, and have a raised shape inclined from centers of the second guide faces 8 in the circumferential direction C toward the third guide faces 9, respectively. Thereby, the respective second guide faces 8 have a shape guiding the needle 61 to the plurality of third guide faces 9.

Here, since the plurality of recess portions 5 are configured with odd-numbered, for example, five recess portions at equal intervals in the circumferential direction C, the plurality of recess portions 5 face the plurality of raised parts 6 of the guiding portion 4, respectively, each of the plurality of raised parts 6 being sandwiched between recess portions 5 in the circumferential direction C of the introduction port 3, with a center of the introduction port 3 sandwiched between each recess portion 5 and a raised part 6 facing the recess portion 5 in the diameter direction K of the introduction port 3. That is, the plurality of recess portions 5 face the third guide faces 9 in the diameter direction K.

Thereby, when the distal end side of the needle 61 whose vibration has been stopped by any of the plurality of recess portions 5 and the plurality of first guide faces 7 is dropped into the treatment instrument insertion assisting tool 1 by a recess portion 5, the distal end side of the needle 61 dropped by the recess portion 5 comes into contact with a third guide face 9 because the third guide face 9 faces the recess portion 5, and the distal end side of the needle 61 can be caught by the third guide face 9.

This is because, if each recess portions 5 faces another recess portion 5 in the diameter direction K, there is a possibility that, when the distal end side of the needle 61 is dropped to the inner side in the diameter direction K via a recess portion 5, the distal end of the needle 61 drops to an outer side of the treatment instrument insertion assisting tool 1 in the diameter direction K via a recess portion 5 facing the recess portion 5.

Note that each recess portion 5 is formed in a semi-elliptical shape or a triangular shape, and each first guide face 7 has a recess shape of inclining toward the center of each first guide face 7 in the circumferential direction C as described above, and this is because the shapes are used to adjust a position where the distal end side of the needle 61 is dropped so that the distal end side of the needle 61 certainly comes into contact with a third guide face 9 when the distal end side of the needle 61 is dropped to the inner side in the diameter direction K via the recess portion 5, by causing the needle 61 caused to come into contact with any one of the first guide faces 7 and a recess portions 5 to be positioned at the center of the first guide face 7 and the recess portion 5 in the circumferential direction C.

Further, even if the distal end side of the needle 61 comes into contact with not a third guide face 9 but a second guide face 8 when the distal end side of the needle 61 is dropped to the inner side in the diameter direction K via a recess portion 5, the distal end side of the needle 61 is guided to a third guide faces 9 because the second guide face 8 has a raised shape inclined from a center of the second guide face 8 toward the third guide face 9.

Furthermore, the part 4nb of the inner circumferential face 4n having the third guide face 9 of each raised part 6 is formed in a saucer shape, and each third guide face 9 is sandwiched by two second guide faces 8 in the circumferential direction C as shown in Fig. 1. Therefore, it is prevented that the distal end side of the needle 61 guided to a third guide face 9 drops to the side of the recess portions 5 positioned on the outer side in the circumferential direction C.

From the above reasons, the plurality of recess portions 5 are configured with odd-numbered, for example, five recess portions. Therefore, the number of recess portions 5 may be any odd number. If the number of recess portions 5 is too large, however, a width of each raised part 6 in the circumferential direction C, that is, a width of each third guide face 9 becomes narrow, and it becomes difficult to catch the distal end side of the needle 61 dropped by a recess portion 5, and it is not preferable. Therefore, actually, it is preferable that the recess portions 5 are configured with three, five or seven recess portions.

Note that the number of recess portions 5 may be one. In this case, however, the work of the surgeon causing the distal end side of the needle 61 to come into contact with a recess portions 5 becomes more difficult than the case where there are three or more recess portions 5 because he must aim the distal end side of the needle 61 at the only one recess portion 5. Therefore, it is not preferable.

Next, configurations of the obturator 50 and an ultrasound probe 100 will be simply described. As shown in Fig. 2, the obturator 50 is provided with the elongated insertion portion 30 and the assisting tool connecting portion 20 provided on a proximal end of the insertion portion 30 in the direction N.

The assisting tool connecting portion 20 is a member to which the connection portion 2 of the treatment instrument insertion assisting tool 1 is connected as described above.

The insertion portion 30 is internally provided with the channel 31 whose distal end and proximal end in the direction N are opened. As shown in Fig. 3, the needle 61 of the biopsy needle 60 is inserted into the channel 31 via the introduction port 3 for the treatment instrument insertion assisting tool 1 and the connection portion 2.

Note that, as shown in Fig. 3, the insertion portion 30 is formed in such a length that, in a state that the needle 61 is inserted into the channel 31, and a distal end of the operation portion 62 is caused to come close to a vicinity of the introduction port 3 for the treatment instrument insertion assisting tool 1, the distal end side of the needle 61 projects forward from a distal end of the insertion portion 30 in the direction N. The length of the projection of the distal end side of the needle 61 from the distal end of the insertion portion 30 becomes freely variable by causing the length of the needle 61 fixed to the operation portion 62 to be variable.

As shown in Fig. 5, the insertion portion 30 is inserted, for example, into a channel 104 of the ultrasound probe 100.

As shown in Fig. 4, the ultrasound probe 100 is provided with an insertion portion 101 having an ultrasound transducer 105 on a distal end side in the direction N, an attaching portion 106 provided on a proximal end of the insertion portion 101 in the direction N, a perfusion port 102 provided on the attaching portion 106 and a signal cable drawing-out portion 103 branching from the attaching portion 106, which constitute main portions of the ultrasound probe 100.

The insertion portion 101 is, for example, inserted into a urethra at time of performing a biopsy procedure for a prostate gland using the biopsy needle 60, and the channel 104 whose distal end and proximal end are opened is formed inside the insertion portion 101.

Note that the insertion portion 30 of the obturator 50 is inserted into the channel 104 as described above. Therefore, a length of the channel 104 in the direction N is shorter than the needle 61 so that the needle 61 projects from a distal end of the channel 104, and the channel 104 is formed in a substantially same length as the insertion portion 30.

Further, in addition to the insertion portion 30 of the obturator 50, an insertion portion of a rigid endoscope not shown and the like can be removably inserted into the channel 104.

Note that the obturator 50 or the rigid endoscope is temporarily fixed to the ultrasound probe 100 by a screw or frictional force or the like after being inserted into the channel 104.

Here, a reason to insert the needle 61 into the channel 104 not directly but via the obturator 50 at time of performing a biopsy procedure for a prostate gland is as follows. The channel 104 is generally formed to have a diameter of about 2.3 mm so that the insertion portion of the rigid endoscope can be also inserted and pulled out as described above. This is because a diameter of an insertion portion of a rigid endoscope with a smallest diameter is about 2 mm at present.

If the needle 61 with a diameter of about 1.2 mm to 1.3 mm is inserted directly into the channel 104, however, backlash of the needle 61 in the diameter direction K occurs in the channel 104. Therefore, a position of the distal end side of the needle 61 projecting from the distal end of the channel 104 is not decided, and it becomes difficult to perform a biopsy procedure.

Therefore, the configuration is used in which the needle 61 is inserted into the channel 31 of the obturator 50 first, and, after that, the insertion portion 30 of the obturator 50 is inserted into the channel 104.

That is, the insertion portion 30 of the obturator 50 is such that eliminates the backlash of the needle 61 by filling a gap between the channel 104 and the needle 61 caused by a difference between the diameter of the channel 104 and the diameter of the needle 61.

Therefore, in the configuration in which it is necessary to insert only the needle 61, without the necessity of inserting the insertion portion of the rigid endoscope into the channel 104, the necessity of using the obturator 50 is eliminated because it is only necessary to form the channel 104 to have a small diameter.

That is, in such a configuration, the connection portion 2 of the treatment instrument insertion assisting tool 1 described above may be configured to be connected to a proximal end of the channel 104. In this case, the ultrasound probe 100 is the tool inserted into a subject.

Further, a configuration is also possible in which the needle 61 is inserted into the channel of the insertion portion of the rigid endoscope not shown instead of the ultrasound probe 100. In such a configuration, the connection portion 2 of the treatment instrument insertion assisting tool 1 described above can be connected to a proximal end of the channel of the rigid endoscope. In this case, the rigid endoscope is the tool inserted into a subject.

The perfusion port 102 communicates with the channel 104. To the perfusion port 102, a perfusion liquid supply source is connected which is for supplying perfusion liquid such as physiological saline solution into a subject via the channel 104 so as to secure a field of view of the rigid endoscope while the insertion portion of the rigid endoscope is inserted into the channel 104 to perform optical observation in the subject.

An extension end of the signal cable drawing-out portion 103 is freely connected to an observation apparatus not shown. Thereby, an ultrasound observed image of an inside of the subject obtained by the ultrasound transducer 105 is displayed on the observation apparatus.

Note that, since other components of the obturator 50 and the ultrasound probe 100 are well known, detailed description of the components will be omitted.

Next, operation of the present embodiment will be simply described.

First, at the time of performing a biopsy procedure for a prostate gland, the surgeon inserts the insertion portion 101 of the ultrasound probe 100 into a urethra and confirms a position of the prostate gland by the ultrasound transducer 105 on an ultrasound observed image. At this time, the surgeon may insert the insertion portion of the rigid endoscope into the channel 104 and confirm the position of the prostate gland while performing optical observation.

Next, after causing the distal end opening of the channel 104 to face a treatment position of the prostate gland, the surgeon inserts the insertion portion 30 of the obturator 50 to which the treatment instrument insertion assisting tool 1 is connected, into the channel 104 until the distal end of the assisting tool connecting portion 20 is positioned near the proximal end opening of the channel 104.

As a result, the distal end of the insertion portion 30 is positioned near the distal end of the channel 104. Note that, in a case of not performing the optical observation, the insertion portion 30 may be inserted into the channel 104 before the insertion portion 101 is inserted into the urethra.

In this state, the surgeon inserts the needle 61 of the biopsy needle 60 into the channel 31 of the obturator 50.

More specifically, in a state of grasping the ultrasound probe 100 by one hand, the surgeon grasps the operation portion 62 of the biopsy needle 60 by the other hand first. As a result, the distal end side of the needle 61 vibrates because the needle 61 of the biopsy needle 60 is elongated and has a small diameter as described above.

Next, the surgeon causes the operation portion 62 to move to cause a part of the needle 61 to come into contact with an edge portion of any one of the plurality of recess portions 5. Note that, at this time, a part of the needle 61 is caused to be along a first guide face 7 also. As a result, the needle 61 comes into contact with the edge portion of any one recess portion 5 and the central part of the first guide face 7 in the circumferential direction C, along the direction N, and vibration of the distal end side stops.

Further, in this state, it does not happen that the needle 61 slides along the circumferential direction C on the outer circumferential edge of the introduction port 3 due to the shapes of the recess portion 5 and the first guide face 7.

After that, the surgeon causes the distal end of the needle 61 whose vibration has stopped to move rearward of the recess portion 5 by causing the operation portion 62 to move rearward in the direction N. As a result, the distal end side of the needle 61 drops to the inner side in the diameter direction K by the recess portion 5 and comes into contact with a third guide face 9.

Note that, at this time, even if the distal end side of the needle 61 comes into contact with the second guide face 8, the distal end side of the needle 61 is guided to the third guide face 9 due to the shape of the second guide face 8 as described above.

Further, at this time, since a raised part 6 having the third guide face 9 has a saucer shape on the part 4nb of the inner circumferential face 4n, and the third guide face 9 is sandwiched between second guide faces 8 in the circumferential direction C, it does not happen that the distal end side of the needle 61 drops off from the third guide face 9 via the recess portion 5 positioned on the outer side in the circumferential direction C.

Lastly, the surgeon presses the operation portion 62 forward in the direction N in the state that the distal end of the needle 61 is in contact with the third guide face 9. As a result, the needle 61 is inserted into the channel 31 via the connection portion 2, and the distal end side of the needle 61 is projected into the prostate gland via each of the distal end openings of the channels 31 and 104.

By the surgeon operating the operation portion 62 in this state, the needle 61 further projects forward, for example, by about 20 mm, and, thereby, the biopsy procedure for the prostate gland is performed. After the biopsy procedure, the surgeon pulls out the needle 61 with tissue of the prostate gland collected at the distal end via the introduction port 3.

Note that the biopsy procedure is performed for a plurality of positions. That is, the work of inserting the needle 61 into the channel 31 via the treatment instrument insertion assisting tool 1 is performed for each biopsy.

Thus, it has been shown in the present embodiment that the plurality of recess portions 5 each of which has such a shape that gradually increases in width from the connection portion 2 side toward the introduction port 3 side and that is recessed toward the connection portion 2 side are provided on the outer circumferential edge of the introduction port 3 for the treatment instrument insertion assisting tool 1.

According to the above, the surgeon can stop vibration of the distal end side of the needle 61 only by causing a part of the needle 61 of the biopsy needle 60 to come into contact with the edge portion of any one of the plurality of recess portions 5 and can certainly prevent the needle 61 from slipping off in the circumferential direction C along the outer circumferential edge of the introduction port 3 by the recess portion 5.

Further, since the distal end of the needle 61 drops into the treatment instrument insertion assisting tool 1 via the recess portion 5 and comes into contact with the inner circumferential face 4n of the guiding portion 4 only by causing the needle 61 to move rearward in the state that the part of the needle 61 is caused to be in contact with the edge portion of the recess portion 5, it is possible to, after that, easily insert the needle 61 into the channel 31 of the obturator 50 only by pressing in the needle 61.

Further, it has been shown in the present embodiment that, since the plurality of odd-numbered recess portions 5 are provided on the outer circumferential edge of the introduction port 3 at equal intervals, the recess portions 5 face the raised parts 6 in the diameter direction K.

According to the above, when vibration of the distal end side of the needle 61 is stopped by a recess portion 5, and the distal end of the needle 61 is dropped to the inner circumferential face 4n by pulling the needle 61 rearward, the distal end side of the needle 61 necessarily comes into contact with a third guide face 9 provided on a part 4nb of the inner circumferential face 4n, of a raised part 6. Therefore, it does not happen that the needle 61 slips off outside the treatment instrument insertion assisting tool 1.

From the above, it is possible to provide a treatment instrument insertion assisting tool 1 having such a configuration that, at time of inserting a treatment instrument into a channel of a tool inserted into a subject to perform treatment at a plurality of positions, the surgeon can easily cause the treatment instrument to be inserted into the channel of the tool inserted into a subject by himself.

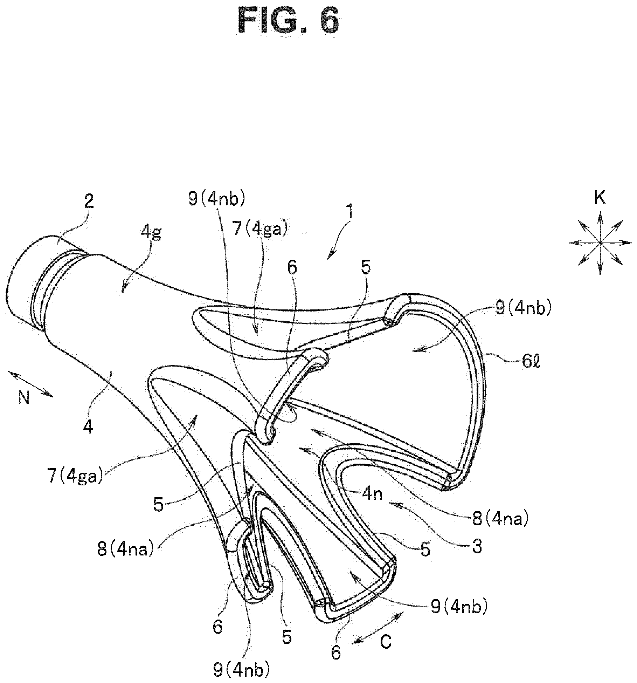

Note that a modification will be described below with use of Fig. 6. Fig. 6 is a perspective view showing a modification of the treatment instrument insertion assisting tool in which the recess portions of Fig. 1 are configured in an even number and provided at unequal intervals, together with the assisting tool connecting portion of the tool inserted into a subject.

It has been shown in the present embodiment described above that, since the plurality of recess portions 5 are configured in an odd number and provided at equal intervals in the circumferential direction C, the plurality of recess portions 5 face the plurality of raised parts 6 of the guiding portion 4, respectively, each of the plurality of raised parts 6 being sandwiched between recess portions 5 in the circumferential direction C of the introduction port 3, with the center of the introduction port 3 sandwiched between each recess portion 5 and a raised part 6 facing the recess portion 5 in the diameter direction K.

This is not limiting. As shown in Fig. 6, by forming only one raised part 61, among the plurality of raised parts 6, to be long in the circumferential direction C, the plurality of recess portions 5 may be configured with even-numbered, for example, four recess portions and formed at unequal intervals in the circumferential direction C.

According to such a configuration, the raised parts 6 face the recess portions 5 in the diameter direction K, and, therefore, effects similar to those of the embodiment described above can be obtained. Note that other effects are similar to those of the embodiment described above.

### (Second embodiment)

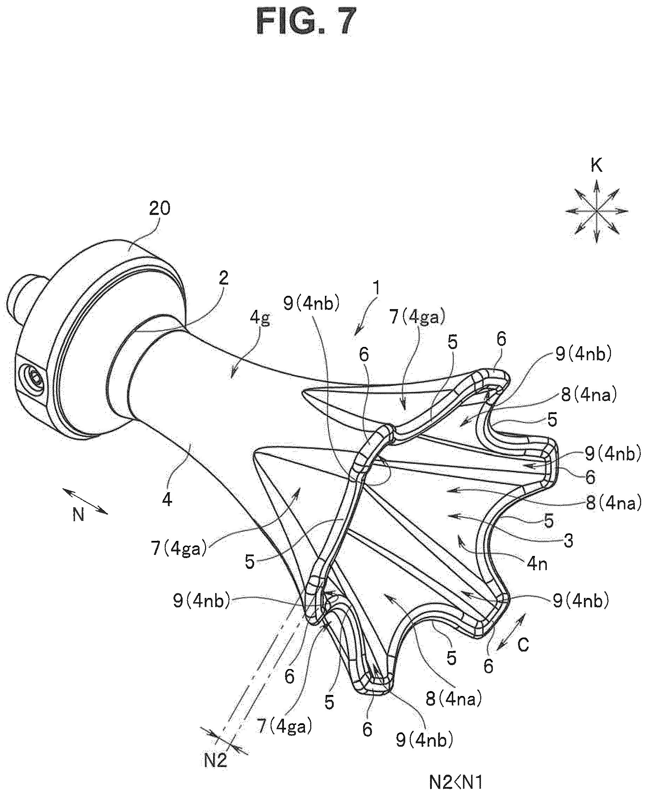

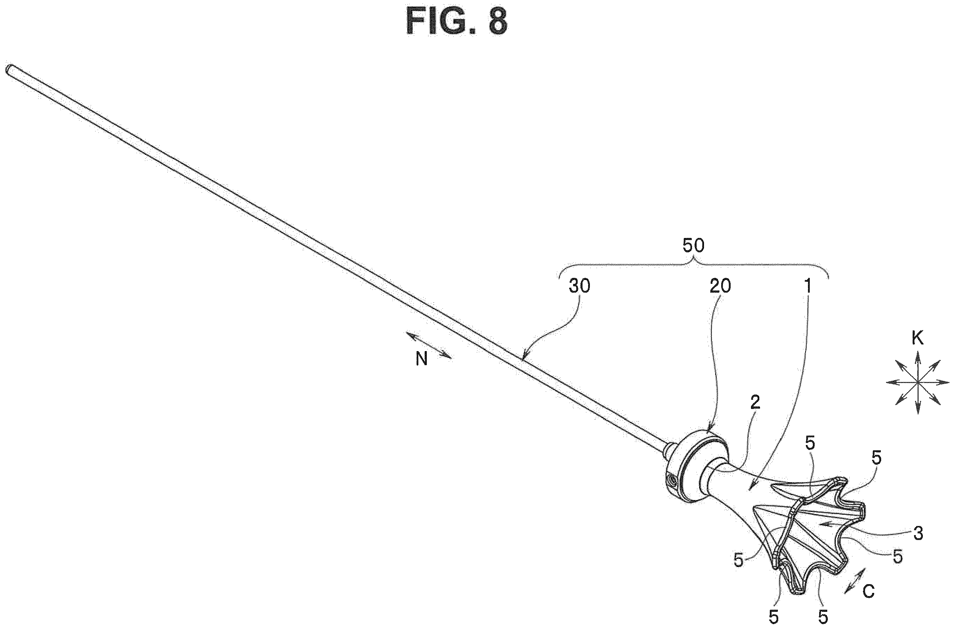

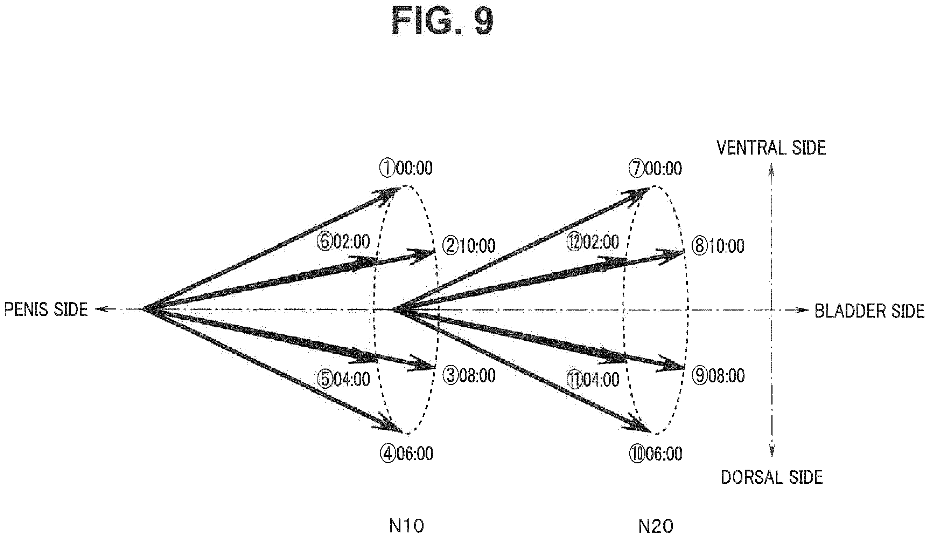

Fig. 7 is a perspective view showing a treatment instrument insertion assisting tool of the present embodiment together with the assisting tool connecting portion of the tool inserted into a subject; Fig. 8 is a perspective view showing the tool inserted into a subject to which the treatment instrument insertion assisting tool of Fig. 7 is connected; and Fig. 9 is a diagram schematically showing positions of puncture with a needle in a case of performing a biopsy procedure for a prostate gland at twelve positions.

In comparison with the treatment instrument insertion assisting tool of the first embodiment shown in Figs. 1 to 5 described above, a configuration of the treatment instrument insertion assisting tool of the second embodiment is different in a length of a recess of a plurality of recess portions and in a point that the plurality of recess portions are configured with six recess portions.

Therefore, only the different points will be described, and components similar to those of the first embodiment will be given same reference numerals, and description of the components will be omitted.

As shown in Figs. 7 and 8, in the present embodiment, the plurality of recess portions 5 provided on the outer circumferential edge of the introduction port 3 for the treatment instrument insertion assisting tool 1 have such a shape that gradually increases in width from the connection portion 2 side toward the introduction port 3 side in the direction N and that is recessed toward the connection portion 2 side by N2 (N2<N1).

Further, as the plurality of recess portions 5, six recess portions are provided on the outer circumferential edge of the introduction port 3, and the six recess portions 5 are provided at equal intervals of 60° in the circumferential direction C of the introduction port 3.

Therefore, in the present embodiment, each recess portion 5 faces another recess portion 5 with the center of the introduction port 3 sandwiched between the recess portions 5 in the diameter direction K.

Note that other components are similar to those of the first embodiment described above.

Next, operation of the present embodiment will be described. Note that description of same operation as the first embodiment will be omitted.

At the time of inserting the needle 61 of the biopsy needle 60 into the channel 31 of the obturator 50, the surgeon causes the operation portion 62 to move and causes a part of the needle 61 to come into contact with the edge portion of any one of the plurality of recess portions 5.

Note that, at this time, a part of the needle 61 is caused to be along a first guide face 7 also. As a result, the needle 61 comes into contact with the edge portion of any one recess portion 5 and the central part of the first guide face 7 in the circumferential direction C, along the direction N, and vibration of the distal end side stops.

Further, in this state, it does not happen that the needle 61 slides along the circumferential direction C on the outer circumferential edge of the introduction port 3 due to the shapes of the recess portion 5 and the first guide face 7.

After that, the surgeon causes the distal end of the needle 61 whose vibration has stopped to move rearward of the recess portion 5 by causing the operation portion 62 to move rearward in the direction N. As a result, the distal end side of the needle 61 drops to the inner side in the diameter direction K by the recess portion 5.

At this time, though each recess portion 5 faces another recess portion 5 in the diameter direction K in the present embodiment, the distal end side of the needle 61 dropped inside the treatment instrument insertion assisting tool 1 in the diameter direction K does not drop outside the treatment instrument insertion assisting tool 1 via a recess portion 5 but drops to a second guide face 8 because the length of the recess portion 5 is extremely short N2. After that, the distal end side of the needle 61 is guided to a third guide faces 9 due to the shape of the second guide face 8.

After that, the surgeon presses the operation portion 62 forward in the direction N in the state that the distal end of the needle 61 is in contact with the third guide face 9. As a result, the needle 61 is inserted into the channel 31 via the connection portion 2, and the distal end side of the needle 61 is projected into the prostate gland via each of the distal end openings of the channels 31 and 104.

By the surgeon operating the operation portion 62 in this state, the needle 61 further projects forward, for example, by about 20 mm, and, thereby, the biopsy procedure for the prostate gland is performed. After the biopsy procedure, the surgeon pulls out the needle 61 with tissue of the prostate gland collected at the distal end via the introduction port 3.

Here, it is common to perform a biopsy procedure for a prostate gland for a plurality of positions as shown in Fig. 9.

More specifically, a method of performing an already-known 12-core biopsy procedure is well known. As shown in Fig. 9, in the 12-core biopsy procedure, each time causing the insertion portion 101 of the ultrasound probe 100 to rotate by 60°, the surgeon causes the needle 61 to project forward to perform an already-known sextant biopsy procedure of performing puncture at six positions on a site N10 of a prostate gland in the direction N, and the surgeon further presses the insertion portion 10 to a bladder side and, by causing the needle 61 forward each time the insertion portion 101 is rotated by 60° on a site N20 of the prostate gland in the direction N, performs the sextant biopsy procedure at six positions again.

Note that the work of inserting the needle 61 into the channel 31 via the treatment instrument insertion assisting tool 1 is performed for each biopsy at one position.

In the 12-core biopsy procedure, each time the insertion portion 101 is rotated by 60°, the obturator 50 inserted into the channel 104 also rotates by 60°, and, therefore, the treatment instrument insertion assisting tool 1 also rotates by 60°.

Here, as described above, the six recess portions 5 are provided on the outer circumferential edge of the introduction port 3 in the present embodiment. That is, a recess portion 5 is provided for each 60°.

Thus, in the case where the surgeon uses the method of causing a part of the needle 61 to come into contact with the edge portion of a recess portion 5, for example, from upward in order to stop vibration of the distal end side of the needle 61, at the time of a biopsy procedure for each position, a next recess portion 5 is not oriented upward when the treatment instrument insertion assisting tool 1 is rotated by 60° in the state that the recess portion is oriented upward, because the number of recess portions 5 is five in the first embodiment described above. Therefore, it becomes difficult to perform the work of causing the needle 61 to come into contact with the recess portion 5, and, furthermore, the surgeon has to perform the rotation by 60° based on the surgeon's sense.

In the present embodiment, however, since the six recess portions 5 are provided, and each recess portion is provided for each 60°, the next recess portion 5 is necessarily positioned on an upper side even when the treatment instrument insertion assisting tool 1 is rotated by 60°.

Therefore, it becomes easy to perform the work of stopping vibration of the distal end side of the needle 61. Additionally, if, in the state that a recess portion 5 is oriented upward before rotation, an adjoining recess portion 5 is oriented, for example, upward after the rotation, the surgeon can easily recognize that rotation by 60° has been performed.

As described above, in the configuration of the present embodiment also, effects similar to those of the first embodiment described above can be obtained, and, additionally, the 12-core biopsy procedure can be performed more easily than in the first embodiment.

Note that, though the biopsy needle 60 has been shown as an example of a treatment instrument in the first and second embodiments described above, the treatment instrument is not limited to that. The treatment instrument may be, of course, a suction tube, a probe or the like, which is an elongated rigid treatment instrument with a small diameter used for other surgical fields and which is basically made of metal and has rigidity and, further, may be a flexible treatment instrument such as a tube.

Further, it has been shown in the first and second embodiment described above that the treatment instrument insertion assisting tool 1 is formed with resin. In this case, though there is an advantage that processing becomes easy at time of forming the treatment instrument insertion assisting tool, there is also a disadvantage that the needle tip of the needle 61 is easily caught on an inner circumferential surface of the treatment instrument insertion assisting tool 1, so that it becomes difficult to perform a biopsy procedure.

Therefore, the treatment instrument insertion assisting tool 1 may be formed with metal. Note that, as the metal, material having an especially high degree of hardness and toughness is preferable. As an example, austenitic stainless steel (JIS SUS304) is given.

As a processing method in the case of using metal, milling by machining, casting such as lost-wax casting, sintering such as MIM sintering, press working of stainless steel plate and the like are given.

More particularly, free-cutting stainless steel (JIS SUS303) which includes sulfur (S) and selenium (Se) is not appropriate because the free-cutting stainless steel is easily bitten by an edged tool and is easy to process, and, therefore, the needle tip of the needle 61 is easily caught.

Note that the free-cutting stainless steel can be used if surface smoothing processing is performed by buffing polishing, chemical polishing or the like.

If the treatment instrument insertion assisting tool 1 is formed with metal as described above, the material is especially superior in toughness, the needle tip of the needle 61 is not easily caught even if it comes into contact with the metal. Further, since the degree of hardness is lower than that of the needle 61, the metal does not damage the needle tip of the needle 61.

Further, a most part of the treatment instrument insertion assisting tool 1 may be made of resin or metal with a low degree of hardness, with priority put on easiness of processing and casting.

More specifically, in the case of forming the treatment instrument insertion assisting tool 1 with resin, a film of metal Ni is formed on a resin surface by electroless nickel plating, and, furthermore, rigid chrome (CR) plating is performed on the film of metal Ni.

In the case of forming the treatment instrument insertion assisting tool 1 with metal, the treatment instrument insertion assisting tool 1 is configured by aluminum (Al) or magnesium (Mg) die casting or lost wax casting.

Note that, in the case of magnesium, thixomolding may be adopted. In this case, chrome plating is performed after electroless nickel plating, similarly to the case of being made of resin. Though a chrome layer on a surface is rigid, the needle tip of the needle 61 is not easily caught because the surface is smooth in comparison with milling processing.

According to such a configuration, processing and forming of the treatment instrument insertion assisting tool 1 becomes easier in comparison with stainless steel described above.

Furthermore, in the first and second embodiments described above, the configuration in which the second guide faces 8 and the third guide faces 9 are provided on the treatment instrument insertion assisting tool 1 has been shown as an example. The configuration is not limiting. Even a configuration in which only the plurality of recess portions 5 are formed at a minimum can prevent vibration of the distal end side of the needle 61 and prevent the distal end side of the needle 61 from slipping off along the circumferential direction C on the outer circumferential edge of the introduction port 3.

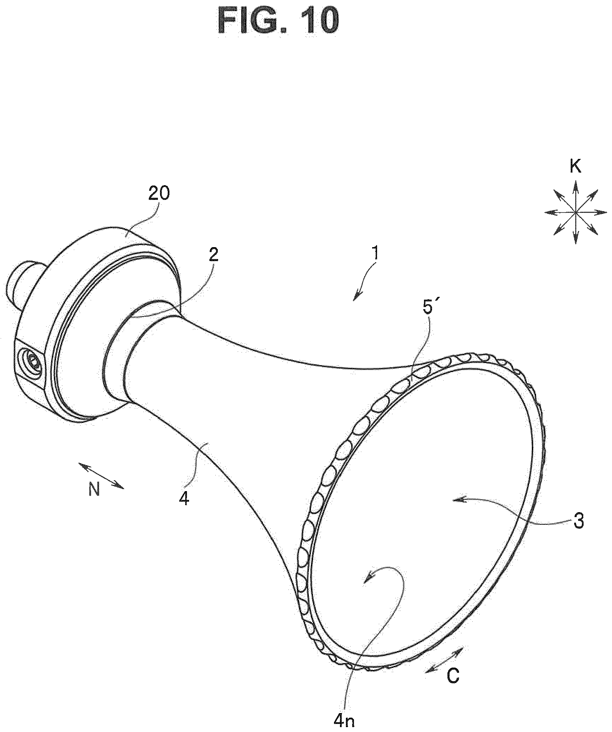

Note that a modification will be described below with use of Fig. 10. Fig. 10 is a perspective view showing a modification of the plurality of recess portions of the treatment instrument insertion assisting tool together with the assisting tool connecting portion of the tool inserted into a subject.

In the first and second embodiments described above, each of the plurality of recess portions 5 formed on the outer circumferential edge of the introduction port 3 for the treatment instrument insertion assisting tool 1 has such a shape that gradually increases in width from the connection portion 2 side toward the introduction port 3 side and that is recessed toward the connection portion 2 side, that is, the plurality of recess portions 5 are recessed by the length N1 or N2 in the direction N.

This is not limiting. As shown in Fig. 10, a plurality of recess portions 5' may be formed only on the outer circumferential edge of the introduction port 3 without extending in the direction N and without communicating with the inner circumferential face 4n.

As an example, 36 recess portions 5' may be formed such that each recess portion 5' is formed for each 10° relative to the outer circumferential edge of the introduction port 3 so as to have a circular arc bottom face having a radius of curvature which is as about twice as an outer shape of the needle 61. Note that a size of the bottom face of the recess portion 5' and the number of recess portions 5' can be appropriately selected.

Thus, in the configuration shown in Fig. 10 also, by a part of the needle 61 being caused to come into contact with a recess portion 5', the recess portion 5' can stop vibration of the distal end side of the needle 61 similarly to the recess portions 5 of the first and second embodiments described above, and it is possible to prevent the needle 61 from slipping off along the circumferential direction C on the outer circumferential edge of the introduction port 3 by the plurality of recess portions 5'.

Note that, at time of causing the needle 61 whose distal end side vibration has been stopped to advance into the treatment instrument insertion assisting tool 1 in the configuration shown in Fig. 10, the surgeon performs the work using the slope of the inner circumferential face 4n, by pulling the needle 61 rearward in the direction N so that the distal end of the needle 61 is positioned rearward of the introduction port 3 and, after that, pressing the distal end of the needle 61 into the treatment instrument insertion assisting tool 1 via the introduction port 3. Therefore, it is more difficult to cause the distal end side of the needle 61 to advance into the treatment instrument insertion assisting tool 1 than in the first and second embodiments described above because the shape of the recess portions 5 and 5' cannot be utilized.

However, since the shape of the treatment instrument insertion assisting tool 1 is simpler than the first and second embodiments, it is possible to form the treatment instrument insertion assisting tool 1 by simple machining. Therefore, it can be easily realized to use metal as the material forming the treatment instrument insertion assisting tool 1, and, therefore, it is possible to easily prevent the needle tip of the needle 61 from biting.

This application is filed, claiming priority based on Japanese Patent Application No. 2014-183513 filed to Japan on September 9, 2014, the content of which is incorporated in the specification, Claims and drawings of this application by reference thereto.

## Claims

1. A treatment instrument insertion assisting tool comprising:
a connection portion configured to be connected to a channel of a tool inserted into a subject;
an introduction port for a treatment instrument inserted into the channel via the connection portion;
a cylindrical guiding portion tapered from the introduction port to the connection portion; and
a plurality of recess portions provided on an outer circumferential edge of the introduction port, each of the recess portions having such a shape that gradually increases in width from the connection portion side toward the introduction port side and that is recessed toward the connection portion side.

2. The treatment instrument insertion assisting tool according to claim 1, wherein raised parts of the guiding portion, each of which is sandwiched between the recess portions in a circumferential direction of the introduction port at the introduction port, and the recess portions mutually face, sandwiching a center of the introduction port in a diameter direction of the introduction port.

3. The treatment instrument insertion assisting tool according to claim 2, wherein a number of the recess portions is an odd number equal to or larger than three, and the recess portions are provided at equal intervals in the circumferential direction relative to the introduction port.

4. The treatment instrument insertion assisting tool according to claim 3, wherein the number of the recess portions is five.

5. The treatment instrument insertion assisting tool according to claim 1, wherein the number of the recess portions is six.

6. The treatment instrument insertion assisting tool according to any one of claims 1 to 5, wherein
first guide faces recessed toward an inner side of the guiding portion in a diameter direction and configured to guide the treatment instrument to the recess portions are formed on outer circumferential faces of parts in communication with the recess portions on the guiding portion; and
second guide faces for the treatment instrument raised toward the inner side in the diameter direction are formed on inner circumferential faces of the parts in communication with the recess portions on the guiding portion.

7. The treatment instrument insertion assisting tool according to claim 6, wherein
third guide faces configured to guide the treatment instrument from the introduction port to the connection port are formed on inner circumferential faces of parts in communication with the raised parts, each of the raised parts being sandwiched between the recess portions in the circumferential direction of the introduction port on the guiding portion; and
the second guide faces have such shapes that guide the treatment instrument to the third guide faces.
